# FASCICULE DE BREVET EUROPEEN

(11) **EP 2 230 296 B1**
(45) Date de publication et mention de la délivrance du brevet: **26.08.2015**
(21) Numéro de dépôt: 10305162.9
(22) Date de dépôt: 17.02.2010
(51) Int. Cl.: C12M 1/12

(54) **Dispositif de culture cellulaire ou tissulaire avec adaptateur et ensemble de culture cellulaire ou tissulaire associé.**
Vorrichtung zur Zell- oder Gewebekultur mit Adapter und entsprechende Anordnung zur Zell- oder Gewebekultur
Cell or tissue culture device with adapter and associated cell or tissue culture assembly

(30) Priorité: 18.02.2009 FR 0951063; 03.04.2009 US 166264 P
(43) Date de publication de la demande: 22.09.2010
(73) Titulaire: L'Oréal, 75008 Paris (FR)
(72) Inventeur: Azadiguian, Gayané, 75012, PARIS (FR); Black, Annie, 92300, LEVALLOIS PERRET (FR)
(74) Mandataire: Jacobson, Claude

(56) Documents cités:
- WO-A-94/28111
- WO-A-2004/020571
- WO-A-2006/131123
- US-A- 5 578 492
- US-A- 5 801 055

## Description

La présente invention concerne un dispositif de culture cellulaire ou tissulaire, du type comprenant :
- un support, destiné à obturer au moins partiellement un bac contenant un milieu de culture, le support comprenant un corps délimitant une surface supérieure et au moins une ouverture traversante de réception de nacelle débouchant dans la surface supérieure ;
- au moins une nacelle montée mobile par rapport au support suivant un axe d'introduction entre une position de culture dans laquelle la nacelle est portée par le support en regard de l'ouverture de réception et une position de manipulation, dans laquelle la nacelle est disposée à l'écart du support,
la nacelle comprenant un récipient de culture et au moins un organe d'appui présentant une surface d'appui sur le support.

Un tel dispositif est destiné notamment à la culture in vitro de différents types de cellules et/ou de tissus, en vue d'utiliser ces cultures pour différents tests ou évaluations. Les échantillons de culture cellulaire s'étendent généralement dans deux dimensions, alors que les échantillons de culture tissulaire s'étendent dans trois dimensions.

Un tel dispositif est notamment destiné à produire des échantillons de tissus de cellules cutanées, tels que des échantillons de peau reconstruite.

On connaît de WO 2006/131123 un dispositif du type précité, destiné à être monté sur un bac contenant un milieu de culture liquide.

Le dispositif comprend un support monté sur le bac, et une pluralité de nacelles amovibles présentant chacune un substrat perméable pour la culture de cellules ou de tissus. La culture des cellules est effectuée en disposant les cellules sur la surface de culture et en alimentant ces cellules à travers le substrat grâce au milieu de culture présent dans le bac inférieur.

A cet effet, les nacelles sont disposées sur le support dans une position de culture. Le support et les nacelles sont ensuite montés conjointement sur le bac pour imprégner le substrat perméable de chaque nacelle par le milieu de culture et permettre ainsi la croissance des cellules.

Chaque nacelle étant mobile individuellement par rapport au support, il est possible de charger et/ou de décharger successivement chaque nacelle de manière indépendante, sans avoir à retirer les autres nacelles du support ou du milieu de culture.

Dans WO 2006/131123, chaque nacelle est introduite dans un puits ménagé dans le bac et contenant un milieu de culture distinct. Les dimensions de la nacelle correspondent sensiblement à celles de l'ouverture de réception ménagée dans le support et à celles de l'ouverture du puits délimité dans le bac.

Un tel dispositif ne donne pas entière satisfaction, notamment dès que les dimensions de la nacelle doivent être modifiées pour s'adapter aux différentes conditions de cultures.

Dans ce cas, il est nécessaire de modifier le dispositif pour qu'il présente un support avec des ouvertures adaptées aux dimensions de la nacelle et un bac présentant des puits de dimensions correspondantes. Un tel ensemble est donc coûteux à réaliser.

Un but de l'invention est donc d'obtenir un dispositif de culture cellulaire ou tissulaire capable d'accommoder des nacelles de tailles variées et qui soit néanmoins peu coûteux à réaliser.

A cet effet, l'invention a pour objet un dispositif du type précité, **caractérisé en ce que** le support comprend un adaptateur de nacelle solidaire du corps et présentant une surface de retenue de nacelle, la ou chaque surface d'appui étant appliquée sur la surface de retenue dans la position de culture, l'organe d'appui étant disposé à l'écart de la périphérie de l'ouverture de réception en projection dans un plan perpendiculaire à l'axe d'introduction lorsque sa surface d'appui est appliquée sur la surface de retenue.

Le dispositif selon l'invention s'adapte à des nacelles de section et/ou de hauteurs variées. Le dispositif selon l'invention s'adapte également aux différents moyens de suspension prévus sur les nacelles. Le dispositif selon l'invention permet également d'optimiser les ratios :
- surface de culture cellulaire ou tissulaire en rapport avec la quantité de milieu de culture à incorporer dans le bac ;
- épaisseur de culture cellulaire ou tissulaire en rapport avec la quantité de milieu de culture à incorporer dans le bac.

Le dispositif selon l'invention peut comprendre l'une ou plusieurs des caractéristiques suivantes, prise(s) isolément ou suivant toute(s) combinaison(s) techniquement possible(s) :
- l'organe d'appui, l'adaptateur et le corps délimitent, en projection dans un plan perpendiculaire à l'axe d'introduction, au moins un passage axial dégagé entre l'organe d'appui et le corps dans l'ouverture de retenue ;
- dans la position de culture, la surface délimitée par le contour extérieur de la nacelle est inférieure à 80% de la surface délimitée par l'ouverture de réception, prises en projection dans un plan perpendiculaire à l'axe d'introduction ;
- le contour extérieur de la nacelle, pris en projection dans un plan perpendiculaire à l'axe d'introduction est situé totalement à l'écart du contour intérieur délimitant l'ouverture de réception ;
- l'adaptateur comprend une lunette délimitant une ouverture d'insertion de nacelle, la surface de retenue s'étendant autour de l'ouverture d'insertion, la nacelle étant reçue dans l'ouverture d'insertion dans sa position de culture ;
- le support comprend au moins une colonne de fixation de l'adaptateur sous le corps ;
- le corps délimite une oreille faisant saillie radialement vers l'axe d'introduction, la colonne de fixation faisant saillie à partir de l'oreille ;
- la surface de retenue s'étend en dessous de la surface supérieure du corps lorsque le corps est disposé sur le bac ;
- le corps délimite une surface inférieure par laquelle débouche l'ouverture de réception, la surface de retenue s'étendant en dessous de la surface inférieure du corps lorsque le corps est disposé sur le bac ;
- la nacelle comprend au moins deux organes d'appui espacés angulairement autour de l'axe d'insertion, l'adaptateur comprenant une surface de retenue de chaque organe d'appui ;
- le ou chaque organe d'appui est formé par un rebord perpendiculaire à l'axe d'introduction faisant saillie radialement à l'écart du récipient ; et
- le récipient comprend une paroi de fond portant une surface de culture et une paroi latérale délimitant une ouverture supérieure, la nacelle délimitant au moins un passage d'alimentation de la surface de culture en milieu de culture, distinct de l'ouverture supérieure.

L'invention a également pour objet un ensemble de culture cellulaire ou tissulaire, **caractérisé en ce qu**'il comprend :
- un bac présentant au moins une cavité destinée à recevoir un milieu de culture ; et
- un dispositif tel que défini plus haut, le support étant monté sur le bac pour placer l'ouverture de réception en regard de la cavité.

L'ensemble selon l'invention peut comprendre l'une ou plusieurs des caractéristiques suivantes, prise(s) isolément ou suivant toute(s) combinaison(s) techniquement possible(s) :
- la cavité délimite un puits de réception de milieu de culture, le puits débouchant par une ouverture supérieure de contour analogue au contour de l'ouverture de réception.

L'invention sera mieux comprise à la lecture de la description qui va suivre, donnée uniquement à titre d'exemple, et faite en se référant aux dessins annexés, sur lesquels :
- la Figure 1 est une vue schématique en perspective éclatée d'un premier ensemble de culture selon l'invention, avec une nacelle occupant une position de manipulation ;
- la Figure 2 est une vue de dessus du dispositif de l'ensemble de la Figure 1, la nacelle occupant une position de culture ;
- la Figure 3 est une vue schématique en coupe suivant un plan vertical III-III du dispositif de la Figure 2.

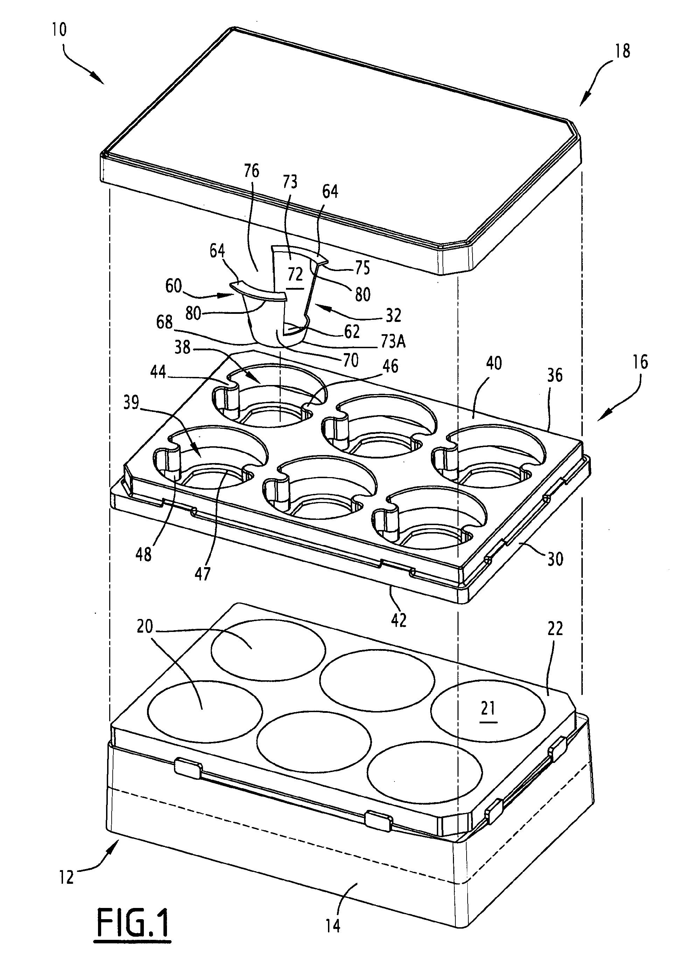

Un premier ensemble de culture 10 selon l'invention est représenté sur les Figures 1 à 3.

Cet ensemble 10 est destiné à cultiver in vitro différents types de cellules et/ou de tissus ou à maintenir ex-vivo différents tissus, en vue de leur utilisation pour différents tests ou évaluations.

Le premier ensemble 10 comprend, de bas en haut sur la Figure 1, un bac 12 de réception d'un milieu de culture 14, un premier dispositif 16 de culture selon l'invention, destiné à être monté sur le bac 12, et un couvercle 18 destiné à coiffer le bac 12 et le dispositif 16, de manière à assurer la stérilité du système. Les échanges gazeux entre l'intérieur du bac 12 recouvert de son couvercle 18 sont autorisés par l'intermédiaire de jours. Toutefois, les jours permettant ces échanges sont de taille telle qu'ils empêchent les contaminations bactériennes.

Le bac 12 délimite au moins une cavité 20 de réception du milieu 14 et une surface supérieure 22 de montage du dispositif 16, s'étendant le long du bord supérieur du bac 12.

Dans l'exemple représenté sur la Figure 1, le bac 12 délimite une pluralité de cavités 20, désignées par le terme « puits », séparées de manière étanche les unes des autres pour contenir différents milieux.

Chaque cavité 20 débouche vers le haut à travers une ouverture supérieure 21 ménagée dans la surface supérieure 22. En variante, le bac 12 présente une cavité 20 unique contenant un milieu de culture 14 commun.

Le milieu 14 est avantageusement un liquide. Il est propre à imprégner les cellules ou tissus à cultiver pour favoriser leur croissance et les maintenir en vie. Le liquide est par exemple une solution aqueuse de différents nutriments tels que des sels minéraux, des réactifs, des vitamines ... Le milieu 14 peut être solide. Dans ce cas, il est dit « gélosé ».

Le dispositif de culture 16 comprend un support de nacelle 30 destiné à être engagé au dessus du bac 12, et au moins une nacelle 32 de culture destinée à être portée par le support de nacelle 30.

Le support 30 comporte un corps 36 sensiblement plat délimitant une pluralité d'ouvertures 38 de réception de nacelles, et, selon l'invention, un adaptateur de nacelle 39 disposé dans ou en regard de chaque ouverture de réception 38.

Dans l'exemple représenté sur la Figure 1, le corps 36 délimite six ouvertures de réception 38. Plus généralement, le nombre d'ouvertures de réception 38 est avantageusement égal au nombre de nacelles 32 et est compris entre 1 et 96.

Le corps 36 s'étend entre une surface supérieure 40 et une surface inférieure 42 de montage sur le bac 12.

Le corps 36 présente avantageusement une épaisseur, prise entre les surfaces 40 et 42 inférieure à ses autres dimensions.

Le corps 36 présente un contour de forme sensiblement complémentaire au contour extérieur du bac 12 le long de son bord supérieur. Dans l'exemple représenté sur la Figure 1, le contour est rectangulaire.

La surface supérieure 40 est sensiblement horizontale lorsque le corps 36 est monté sur le bac 12.

Le corps 40 délimite dans chaque ouverture de réception 38 deux oreilles 44, 46 de maintien de l'adaptateur 39. Les oreilles 44, 46 font saillie radialement dans l'ouverture 38 vers l'axe d'insertion A-A' de la nacelle 32 dans chaque ouverture. Elles sont situées en regard l'une de l'autre de part et d'autre de l'axe A-A'. Elles s'étendent verticalement sur toute l'épaisseur du corps 36, prise entre la surface supérieure 40 et la surface inférieure 42.

Chaque ouverture de réception 38 s'étend le long de l'axe vertical A-A' d'insertion de la nacelle dans l'ouverture 38, entre la surface inférieure 42 et la surface supérieure 40. Chaque ouverture de réception 38 est traversante, de sorte qu'elle débouche dans les surfaces 40 et 42.

Chaque ouverture 38 présente une section horizontale sensiblement constante sur toute sa hauteur à travers le corps 36. Cette section est analogue à la section de l'ouverture supérieure 21.

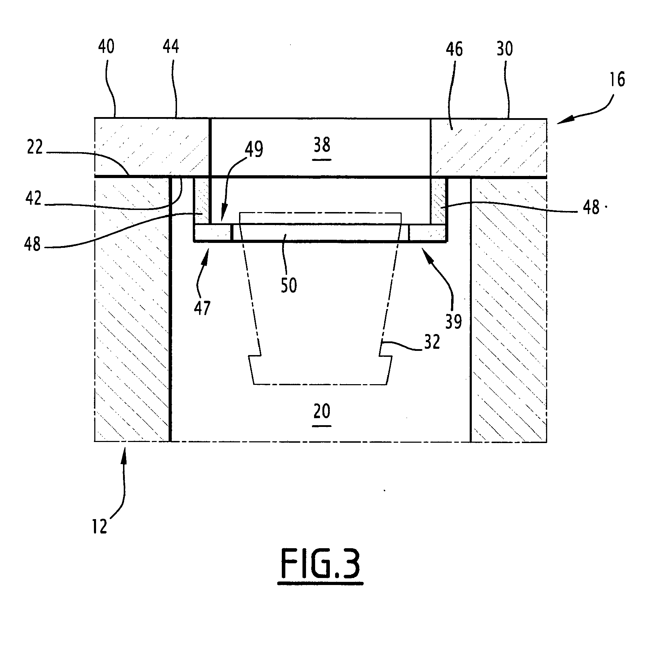

Comme illustré par la Figure 3, l'adaptateur de nacelle 39 comprend une lunette 47 de support de la nacelle et des colonnes 48 de fixation de la lunette sous le corps.

Dans cet exemple, une lunette 47 est disposée en regard de chaque ouverture de réception 38. Chaque lunette 47 est formée par un cadre ajouré mince, d'épaisseur inférieure à celle du corps 36. La lunette 47 délimite une surface supérieure 49 sensiblement plane de retenue de la nacelle et une ouverture centrale 50 d'insertion de la nacelle 32.

Dans cet exemple, en référence à la figure 3, la surface de retenue 49 est située en dessous de la surface inférieure 42 du corps 36. Elle s'étend dans un plan perpendiculaire à l'axe A-A'

La lunette 47 présente un contour extérieur de forme sensiblement ovale et un contour intérieur fermé délimitant l'ouverture centrale 50.

En projection dans un plan perpendiculaire à l'axe A-A', le contour extérieur de la surface de retenue 49 est situé à l'écart du contour intérieur de l'ouverture de réception 28 de nacelle.

Ainsi, en projection dans ce plan, l'adaptateur de nacelle 39 et le corps 36 délimitent en regard de l'ouverture de réception 38, deux passages axiaux 51 en forme générale de C s'étendant de part et d'autre d'un plan passant par les oreilles 44, 46.

La surface délimitée autour de l'axe A-A' par le contour extérieur de la lunette 47 est inférieure à 80% de la surface délimitée autour de l'axe A-A' par le contour intérieur de l'ouverture de réception 38.

La surface de retenue 49 comprend une première région 52 et une deuxième région 53 s'étendant respectivement de part et d'autre d'un plan vertical médian de l'ouverture de réception 38 passant par les oreilles 44, 46.

Les colonnes de fixation 48 s'étendent sous les oreilles 44, 46. Chaque colonne 48 comprend une extrémité supérieure fixée sous une oreille 44, 46 et une extrémité inférieure fixée sur la surface de retenue 49 de la lunette 47.

Ainsi, les colonnes 48 maintiennent la surface de retenue 49 en dessous de la surface inférieure du corps 42. La lunette 47 est centrée par rapport à l'axe A-A'.

Les surfaces de retenue 49 des différentes lunettes 47 en regard des ouvertures 38 sont avantageusement situées à la même hauteur par rapport au corps 36.

Dans l'exemple représenté sur les Figures, la lunette 47 et les colonnes 48 sont formées de pièces rapportées sur le corps 34.

En variante, le corps 34, la lunette 47, et les colonnes 48 sont venus de matière.

Dans l'exemple représenté sur les Figures 1 à 3, le support 30 est mobile par rapport au bac de base 12 entre une position engagée sur le bac 12, dans laquelle le support 14 obture sensiblement le bac 20 vers le haut, à l'exception des ouvertures de passage 38 placées en regard de chaque cavité 10, et une position démontée à l'écart du bac 12 pour la manipulation conjointe des nacelles 32 retenues dans le support 30, comme on va le décrire en détail plus bas.

Lorsque le support 30 occupe sa position montée sur le bac 12, la surface supérieure de retenue 40 est orientée vers le haut.

Dans l'exemple représenté sur les Figures 1 à 3, chaque nacelle 32 comprend un récipient 60 creux, un substrat de culture 62 rapporté dans le récipient 60, et deux organes d'appui formés par des rebords d'appui 64 destinés à s'appuyer sur la surface de retenue 49. En variante (non représentée), la nacelle comporte trois organes d'appui équirépartis à sa périphérie et se présentant sous forme de bras s'étendant radialement.

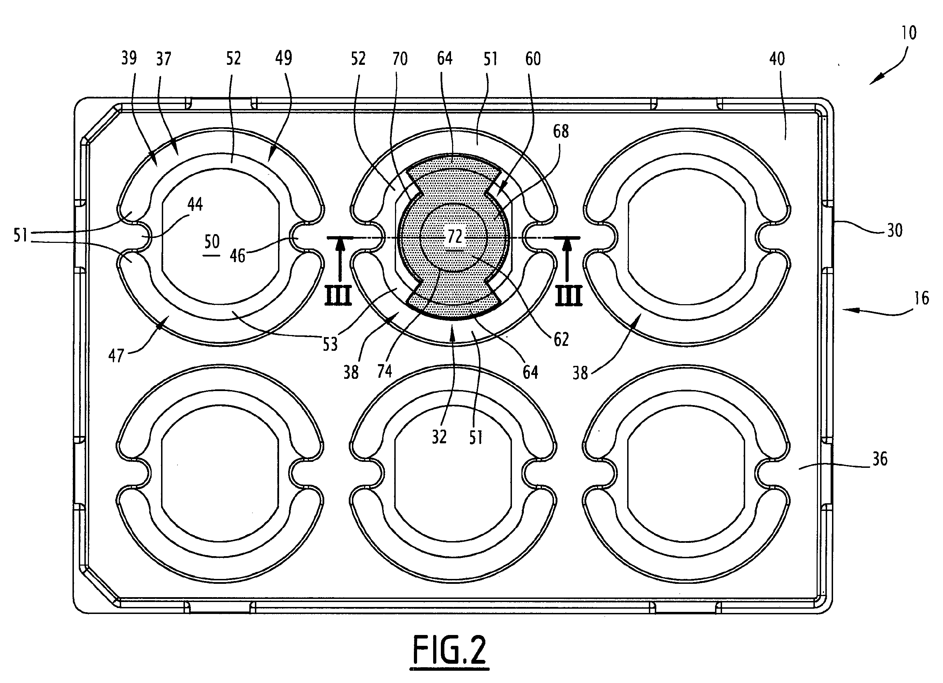

Le récipient 60 comprend une paroi de fond 68 sensiblement horizontale sur la Figure 2 et une paroi périphérique latérale 70, qui délimitent un volume intérieur 72 de culture débouchant par une ouverture supérieure 73.

La jonction 73A entre la paroi de fond 68 et la paroi périphérique latérale 70 est avantageusement arrondie dans le volume intérieur 72, afin d'assurer un écoulement idéal des différents liquides qui sont susceptibles d'être déposés dans le volume intérieur 72.

L'accès au volume intérieur 72 peut être effectué par les passages axiaux 51. Les passages axiaux 51 garantissent ainsi les échanges gazeux entre les différents compartiments du dispositif et entre le contenu de la nacelle 32 et le milieu de culture situé en dessous.

La paroi de fond 68 s'étend sensiblement perpendiculairement à l'axe d'insertion A-A'.

La paroi de fond 68 délimite un passage central 74 d'alimentation en milieu de culture débouchant dans le volume intérieur 72. Dans l'exemple représenté sur les Figures 1 à 3, le passage central 74 est d'axe A-A' et de forme sensiblement circulaire.

Il présente une surface variable en fonction du substrat de culture et généralement supérieure à 10% de la surface délimitée par la paroi de fond.

La paroi latérale 70 fait saillie vers le haut à partir de la paroi de fond 68 jusqu'à un bord supérieur 75. Elle présente une forme sensiblement tronconique d'axe A-A'. Elle délimite deux encoches latérales 76 d'accès au volume intérieur 72 situés de part et d'autre d'un plan vertical médian passant par l'axe A-A'.

Le bord supérieur 75 est de forme sensiblement complémentaire à la forme de l'ouverture d'insertion 50.

Le substrat de culture 62 est rapporté sur la paroi de fond 68 en partie supérieure ou inférieure autour du passage 74. Ce substrat obture le passage 74. Le substrat 62 est perméable pour permettre l'entrée de milieu de culture dans le volume intérieur 72 à travers le passage 74. Il définit une surface supérieure de culture 78 destinée à recevoir les cellules ou tissus à cultiver. Il est réalisé par exemple à base d'une éponge de collagène, ou d'un film de collagène ou à base d'une matrice en polycarbonate, ou d'un tissu synthétique tel que le nylon ou encore d'une grille métallique.

Dans une variante (non représentée), le substrat 62 est formé directement dans la paroi de fond 68 en étant venu de matière avec la paroi de fond. Il délimite une pluralité d'ouvertures de passages 74 pour la diffusion de milieux de culture dans le volume 72.

Dans une autre variante, le substrat 62 est imperméable et au moins un passage d'alimentation 74 est ménagé à l'écart de la surface de culture 78 dans la paroi de fond 68 ou dans une partie inférieure de la paroi latérale 70 située au voisinage de la paroi de fond 68.

Dans l'exemple représenté sur les Figures 1 à 3, chaque rebord d'appui 64 est venu de matière avec le récipient 60. Il s'étend radialement à l'écart de l'axe A-A' perpendiculairement à cet axe A-A'.

Chaque rebord 64 définit une première surface inférieure d'appui 80 destinée à être appliquée respectivement sur la première région 52 ou de la deuxième région 53 de la surface de retenue 49 dans une position de culture de la nacelle.

Chaque nacelle 32 est mobile par rapport au support 30 entre une position de culture, représentée sur les Figures 2 et 3, dans laquelle elle est engagée dans une ouverture de réception 38 et dans une ouverture d'insertion 50 d'un adaptateur 37, et une position de manipulation, représentée sur la Figure 1, dans laquelle la nacelle 32 est disposée à l'écart du support 30 pour être déplaçable librement par rapport au support 30.

Dans la position de culture, la nacelle 32 a été insérée dans une ouverture de réception 38, parallèlement à l'axe A-A', puis dans l'ouverture d'insertion 50 de l'adaptateur 39 correspondant à l'ouverture de réception 38.

Dans cette position, les premières surfaces d'appui 80 sont appliquées respectivement contre la première région 52 et contre la deuxième région 53 de la surface de retenue 49.

Les rebords 64 sont disposés à l'écart de la périphérie de l'ouverture de retenue 38 en projection dans un plan perpendiculaire à l'axe A-A'.

Les passages axiaux 51 sont dégagés en se déplaçant de haut en bas, ce qui permet l'introduction dans la cavité 20 d'un outil de prélèvement, de remplissage ou de dosage, tel qu'une pipette à travers le corps 36, pour alimenter en milieu de culture ou prélever ou ajouter une autre substance dans le milieu de culture, sans avoir à déplacer la nacelle 32.

Les encoches 76 sont disposées en regard respectivement des oreilles 44, 46.

La surface délimitée par la nacelle 32, prise en projection dans un plan horizontal perpendiculaire à l'axe d'insertion A-A' est avantageusement inférieure à 80% de la surface délimitée par le contour intérieur de l'ouverture de réception 38.

La paroi de fond 68 fait saillie vers le bas au delà de la surface inférieure 42 pour être apte à tremper dans le milieu de culture 14 lorsque le support 30 est monté sur le bac 12.

La lunette 47 est dimensionnée pour recevoir la nacelle 32 en fonction de la forme et de la taille de cette nacelle, tout en conservant les dimensions du corps 36, des ouvertures de retenue 38, du bac 12 et du couvercle 18 de taille constante et indépendante de la taille de la nacelle 12.

Il est donc possible de standardiser la fabrication du bac 12, du couvercle 18 et du corps 36 du support 30, ce qui réduit notablement le coût du premier ensemble 10 selon l'invention, tout en conservant la possibilité d'utiliser des nacelles de tailles très différentes avec le même bac 12 et le même couvercle 18.

Le couvercle 18 est propre à coiffer le dispositif 16 et le bac 12 pour délimiter, à l'intérieur de l'ensemble de culture 10, un espace de culture présentant une atmosphère contrôlée et stérile et contenant les nacelles 32.

Le fonctionnement du premier ensemble 10 selon l'invention va maintenant être décrit.

Initialement, chaque nacelle 32 est chargée avec des cellules et/ou un tissu à cultiver.

A cet effet, les cellules et/ou le tissu sont déposés sur la surface de culture 78. Le milieu de culture 14 est par ailleurs introduit dans la cavité 20.

Chaque nacelle 32 est montée dans sa position de culture sur le support 30. A cet effet, chaque récipient creux 60 est introduit dans une ouverture de réception 38 depuis la surface supérieure 40, puis est introduit dans l'ouverture d'insertion 50 délimitée par la lunette 47, jusqu'à ce que chaque surface d'appui 80 des rebords d'appui 64 soit disposée respectivement sur la première région 52 et sur la deuxième région 53 de la surface de retenue 49.

La nacelle 32 est alors retenue verticalement le long de l'axe A-A' à l'encontre de son poids par la lunette 47 solidaire du support 30.

Puis, le dispositif de culture 16 est monté sur le bac 12. Lors du passage du dispositif 16 de la position démontée à la position montée, la paroi de fond 68 de chaque nacelle 32 s'immerge dans le milieu de culture 14 pour imbiber les cellules et/ou le tissu à travers le passage d'alimentation 74 et le substrat perméable 62.

Le couvercle 18 est alors refermé pour coiffer le dispositif 16.

En variante, le support 30 est monté sur la bac 12 avant la disposition des nacelles 32 dans les ouvertures de réception 38.

Lorsqu'un prélèvement et/ou un test doit être effectué individuellement sur une nacelle donnée 32, un opérateur déplace la nacelle 32 à l'écart de l'ouverture de réception 38 pour effectuer le prélèvement et/ou le test, tout en maintenant éventuellement le support 30 monté sur le bac 12.

Au contraire, lorsque plusieurs nacelles 32 d'un groupe de nacelles chargées sur le support 30 doivent être manipulées ensemble, le support 30 et les nacelles 32 dans leurs positions de culture sont déplacés conjointement à l'écart du bac 12.

En outre, lorsque les nacelles 32 sont retenues dans le support 30, il est possible de les mettre en contact simultanément avec un seul réactif ou encore d'effectuer un traitement thermique, par exemple une congélation par contact.

Dans ce dernier cas, les parois de fond 68 des nacelles 72 dans leurs positions de culture sont avantageusement toutes maintenues dans le même plan horizontal par le support 30.

Même lorsque les nacelles 32 ne sont que posées sur le support 30 sans y être retenues, les substrats 62 peuvent être maintenus dans un même plan horizontal ce qui permet de les placer simultanément au contact d'un réactif ou en position d'interface air-liquide.

Dans une variante (non représentée), la lunette 47 est discontinue sur sa périphérie en se déplaçant autour de l'axe A-A'. Elle délimite ainsi au moins deux parties disjointes, par exemple en forme de C.

Dans une variante (non représentée), le support 30 est muni de moyens de retenue libérables de chaque nacelle 32 dans la position de culture formés par exemple par un organe de retenue solidaire de la nacelle et par un organe de retenue complémentaire coopérant par enserrement, par vissage, par butée, ou/et par encliquetage avec l'organe de retenue afin de permettre le retournement conjoint du support 30 et de la nacelle 32 pour placer la surface supérieure 40 orientée vers le bas tout en maintenant la nacelle 32 solidaire du support 30.

Avantageusement, les ensembles selon l'invention sont réalisés au format SBS pour permettre une manipulation par un robot.

## Revendications

1. Dispositif (16) de culture cellulaire ou tissulaire, du type comprenant :
- un support (30), destiné à obturer au moins partiellement un bac (12) contenant un milieu de culture (14), le support (30) comprenant un corps (36) délimitant une surface supérieure (40) et au moins une ouverture traversante (38) de réception de nacelle débouchant dans la surface supérieure (40) ;
- au moins une nacelle (32) montée mobile par rapport au support (30) suivant un axe d'introduction (A-A') entre une position de culture dans laquelle la nacelle (32) est portée par le support (30) en regard de l'ouverture de réception (38) et une position de manipulation, dans laquelle la nacelle (32) est disposée à l'écart du support (30),
la nacelle (32) comprenant un récipient (60) de culture et au moins un organe d'appui (64) présentant une surface d'appui (80) sur le support,
**caractérisé en ce que** le support (30) comprend un adaptateur (39) de nacelle solidaire du corps (36) et présentant une surface (49) de retenue de nacelle, la ou chaque surface d'appui (80) étant appliquée sur la surface de retenue (49) dans la position de culture, l'organe d'appui (64) étant disposé à l'écart de la périphérie de l'ouverture de réception (38) en projection dans un plan perpendiculaire à l'axe d'introduction (A-A') lorsque sa surface d'appui (80) est appliquée sur la surface de retenue (49).

2. Dispositif (16) selon la revendication 1, **caractérisé en ce que** l'organe d'appui (64), l'adaptateur (39) et le corps (36) délimitent, en projection dans un plan perpendiculaire à l'axe d'introduction (A-A'), au moins un passage axial (51) dégagé entre l'organe d'appui (64) et le corps (36) dans l'ouverture de retenue (38).

3. Dispositif (16) selon l'une quelconque des revendications précédentes, **caractérisé en ce que** dans la position de culture, la surface délimitée par le contour extérieur de la nacelle (32) est inférieure à 80% de la surface délimitée par l'ouverture de réception (38), prises en projection dans un plan perpendiculaire à l'axe d'introduction (A-A').

4. Dispositif (16) selon l'une quelconque des revendications précédentes, **caractérisé en ce que** le contour extérieur de la nacelle (32), pris en projection dans un plan perpendiculaire à l'axe d'introduction (A-A') est situé totalement à l'écart du contour intérieur délimitant l'ouverture de réception (38).

5. Dispositif (16) selon l'une quelconque des revendications précédentes, **caractérisé en ce que** l'adaptateur (39) comprend une lunette (47) délimitant une ouverture (50) d'insertion de nacelle, la surface de retenue (49) s'étendant autour de l'ouverture d'insertion (50), la nacelle (32) étant reçue dans l'ouverture d'insertion (50) dans sa position de culture.

6. Dispositif (16) selon l'une quelconque des revendications précédentes, **caractérisé en ce que** le support (30) comprend au moins une colonne (48) de fixation de l'adaptateur (39) sous le corps (36).

7. Dispositif (16) selon la revendication 6, **caractérisé en ce que** le corps (36) délimite une oreille (46) faisant saillie radialement vers l'axe d'introduction (A-A'), la colonne de fixation (48) faisant saillie à partir de l'oreille (46).

8. Dispositif (16) selon l'une quelconque des revendications précédentes, **caractérisé en ce que** la surface de retenue (49) s'étend en dessous de la surface supérieure du corps (36) lorsque le corps (36) est disposé sur le bac (12).

9. Dispositif (16) selon la revendication 8, **caractérisé en ce que** le corps (36) délimite une surface inférieure (42) par laquelle débouche l'ouverture de réception (38), la surface de retenue (49) s'étendant en dessous de la surface inférieure du corps (36) lorsque le corps (36) est disposé sur le bac (12).

10. Dispositif (16) selon l'une quelconque des revendications précédentes, **caractérisé en ce que** la nacelle (32) comprend au moins deux organes d'appui (64) espacés angulairement autour de l'axe d'insertion (A-A'), l'adaptateur (39) comprenant une surface de retenue (49) de chaque organe d'appui (64).

11. Dispositif (16) selon l'une quelconque des revendications précédentes, **caractérisé en ce que** le ou chaque organe d'appui (64) est formé par un rebord perpendiculaire à l'axe d'introduction (A-A') faisant saillie radialement à l'écart du récipient (60).

12. Dispositif (16) selon l'une quelconque des revendications précédentes, **caractérisé en ce que** le récipient (60) comprend une paroi de fond (68) portant une surface de culture et une paroi latérale (70) délimitant une ouverture supérieure (73), la nacelle (32) délimitant au moins un passage (74) d'alimentation de la surface de culture en milieu de culture, distinct de l'ouverture supérieure (73).

13. Ensemble (10) de culture cellulaire ou tissulaire, **caractérisé en ce qu'**il comprend :
- un bac (12) présentant au moins une cavité (20) destinée à recevoir un milieu de culture (14);
- un dispositif (16) selon l'une quelconque des revendications précédentes, le support (30) étant monté sur le bac (12) pour placer l'ouverture de réception (38) en regard de la cavité (20).

14. Ensemble (10) selon la revendication 13, **caractérisé en ce que** la cavité (20) délimite un puits de réception de milieu de culture, le puits débouchant par une ouverture supérieure (21) de contour analogue au contour de l'ouverture de réception (38).

## Patentansprüche

1. Zell- oder Gewebekulturvorrichtung (16) des Typs, der umfasst:
- einen Träger (30), der zum mindestens teilweisen Verschließen eines ein Kulturmedium (14) enthaltenden Behälters (12) bestimmt ist, wobei der Träger (30) einen eine obere Fläche (40) begrenzenden Körper (36) und mindestens eine durchgehende Näpfchenaufnahmeöffnung (38) umfasst, die in die obere Fläche (40) ausmündet,
- mindestens ein im Verhältnis zum Träger (30) gemäß einer Einführachse (A-A') zwischen einer Kulturposition, in welcher das Näpfchen (32) von dem Träger (30) gegenüber der Aufnahmeöffnung (38) gehalten wird, und einer Handhabungsposition, in welcher das Näpfchen (32) vom Träger (30) beabstandet angeordnet ist, bewegbar montiertes Näpfchen (32),
wobei das Näpfchen (32) eine Kulturbehältnis (60) und mindestens ein Stützelement (64) umfasst, das eine Stützfläche (80) auf dem Träger aufweist,
**dadurch gekennzeichnet, dass** der Träger (30) einen mit dem Körper (36) fest verbundenen Näpfchenadaptater (39) umfasst, der eine Näpfchenhaltefläche (49) aufweist, wobei die oder jede Stützfläche (80) in der Kulturposition auf der Haltefläche (49) anliegt, wobei das Stützelement (64), wenn seine Stützfläche (80) auf der Haltefläche (49) anliegt, in Projektion in einer zur Einführachse (A-A') senkrechten Ebene beabstandet vom Umfang der Aufnahmeöffnung (38) angeordnet ist.

2. Vorrichtung (16) nach Anspruch 1, **dadurch gekennzeichnet, dass** das Stützelement (64), der Adapter (39) und der Körper (36) in Projektion in einer zur Einführachse (A-A') senkrechten Ebene mindestens einen zwischen dem Stützelement (64) und dem Körper (36) freien axialen Durchgang (51) in der Halteöffnung (38) begrenzen.

3. Vorrichtung (16) nach einem der vorangehenden Ansprüche, **dadurch gekennzeichnet, dass**, herangezogen in Projektion in einer zur Einführachse (A-A') senkrechten Ebene, die in der Kulturposition von der Außenkontur des Näpfchens (32) begrenzte Fläche kleiner als 80 % als die von der Aufnahmeöffnung (38) begrenzte Fläche ist.

4. Vorrichtung (16) nach einem der vorangehenden Ansprüche, **dadurch gekennzeichnet, dass**, herangezogen in Projektion in einer zur Einführachse (A-A') parallelen Ebene, sich die Außenkontur des Näpfchens (32) vollkommen von der die Aufnahmeöffnung (38) begrenzenden Innenkontur beabstandet befindet.

5. Vorrichtung (16) nach einem der vorangehenden Ansprüche, **dadurch gekennzeichnet, dass** der Adapter (39) einen Reif (47) umfasst, der eine Näpfcheneinsetzöffnung (50) begrenzt, wobei sich die Haltefläche (49) um die Einsetzöffnung (50) erstreckt, wobei das Näpfchen (32) in seiner Kulturposition in der Einsetzöffnung (50) aufgenommen ist.

6. Vorrichtung (16) nach einem der vorangehenden Ansprüche, **dadurch gekennzeichnet, dass** der Träger (30) mindestens eine Befestigungssäule (48) des Adapters (39) unter dem Körper (36) umfasst.

7. Vorrichtung (16) nach Anspruch 6, **dadurch gekennzeichnet, dass** der Körper (36) ein Ohr (46) begrenzt, das radial zur Einführachse (A-A') absteht, wobei die Befestigungssäule (48) ab dem Ohr (46) absteht.

8. Vorrichtung (16) nach einem der vorangehenden Ansprüche, **dadurch gekennzeichnet, dass** sich die Haltefläche (49) unter der oberen Fläche des Körpers (36) erstreckt, wenn der Körper (36) auf dem Behälter (12) angeordnet ist.

9. Vorrichtung (16) nach Anspruch 8, **dadurch gekennzeichnet, dass** der Körper (36) eine untere Fläche (42) begrenzt, durch die die Aufnahmeöffnung (38) ausmündet, wobei sich die Haltefläche (49) unter der unteren Fläche des Körpers (36) erstreckt, wenn der Körper (36) auf dem Behälter (12) angeordnet ist.

10. Vorrichtung (16) nach einem der vorangehenden Ansprüche, **dadurch gekennzeichnet, dass** das Näpfchen (32) mindestens zwei winklig um die Einführachse (A-A') beabstandete Stützelemente (64) umfasst, wobei der Adapter (39) eine Haltefläche (49) jedes Stützelementes (64) umfasst.

11. Vorrichtung (16) nach einem der vorangehenden Ansprüche, **dadurch gekennzeichnet, dass** das oder jedes Stützelement (64) von einem zur Einführachse (A-A') senkrecht angeordneten Rand gebildet ist, der radial vom Behältnis (60) beabstandet absteht.

12. Vorrichtung (16) nach einem der vorangehenden Ansprüche, **dadurch gekennzeichnet, dass** das Behältnis (60) eine eine Kulturfläche tragende Bodenwand (68) und eine eine obere Öffnung (73) begrenzende Seitenwand (70) umfasst, wobei das Näpfchen (32) mindestens einen Versorgungsdurchgang (74) der Kulturfläche mit Kulturmedium begrenzt, der sich von der oberen Öffnung (73) unterscheidet.

13. Zell- oder Gewebekultureinheit (10), **dadurch gekennzeichnet, dass** sie umfasst:
- einen Behälter (12), der mindestens einen für die Aufnahme eines Kulturmediums (14) bestimmten Hohlraum (20) aufweist,
- eine Vorrichtung (16) nach einem der vorangehenden Ansprüche, wobei der Träger (30) auf dem Behälter (12) montiert ist, um die Aufnahmeöffnung (38) gegenüber dem Hohlraum (20) zu platzieren.

14. Einheit (10) nach Anspruch 13, **dadurch gekennzeichnet, dass** der Hohlraum (20) eine Kulturmedium-Aufnahmevertiefung begrenzt, wobei die Vertiefung durch eine obere Öffnung (21) mit einer Kontur ausmündet, die zur Kontur der Aufnahmeöffnung (38) analog ist.

## Claims

1. A cell or tissue culture device (16), of the type comprising:
- a support (30), designed to at least partially close off a tray (12) containing the culture medium (14), the support (30) comprising a body (36) delimiting an upper surface (40) and at least one through opening (38) receiving a nacelle emerging in the upper surface (40);
- at least one nacelle (32) mounted movably relative to the support (30) along an insertion axis (A-A') between a culture position in which the nacelle (32) is supported by the support (30) across from the reception opening (38), and a handling position in which the nacelle (32) is positioned separated from the support (30),
the nacelle (32) comprising a culture container (60) and at least one bearing member (64) having a bearing surface (80) on the support,
**characterized in that** the support (30) comprises a nacelle adapter (39) secured with the body (36) and having a nacelle retaining surface (49), the or each bearing surface (80) being pressed on the retaining surface (49) in the culture position, the bearing member (64) being positioned away from the periphery of the receiving opening (38) projected in a plane perpendicular to the insertion axis (A-A') when its bearing surface (80) is pressed on the retaining surface (49).

2. The device (16) according to claim 1, **characterized in that** the bearing member (64), the adapter (39) and the body (36) delimit, projected in a plane perpendicular to the insertion axis (A-A'), at least one axial passage (51) freed between the bearing member (64) and the body (36) in the retaining opening (38).

3. The device (16) according to any one of the preceding claims, **characterized in that** in the culture position, the surface delimited by the outer contour of the nacelle (32) is less than 80% of the surface delimited by the reception opening (38), projected in a plane perpendicular to the insertion axis (A-A').

4. The device (16) according to any one of the preceding claims, **characterized in that** the outer contour of the nacelle (32), projected in a plane perpendicular to the insertion axis (A-A'), is situated completely separated from the inner contour delimiting the reception opening (38).

5. The device (16) according to any one of the preceding claims, **characterized in that** the adapter (39) comprises a bezel (47) delimiting a nacelle insertion opening (50), the retaining surface (49) extending around the insertion opening (50), the nacelle (32) being received in the insertion opening (50) in its culture position.

6. The device (16) according to any one of the preceding claims, **characterized in that** the support (30) comprises at least one column (48) for fastening the adapter (39) below the body (36).

7. The device (16) according to claim 6, **characterized in that** the body (36) delimits a lug (46) protruding radially toward the insertion axis (A-A'), the fastening column (48) protruding from the lug (46).

8. The device (16) according to any one of the preceding claims, **characterized in that** the retaining surface (49) extends below the upper surface of the body (36) when the body (36) is positioned on the tray (12).

9. The device (16) according to claim 8, **characterized in that** the body (36) delimits a lower surface (42) through which the receiving opening (38) emerges, the retaining surface (49) extending below the lower surface of the body (36) when the body (36) is positioned on the tray (12).

10. The device (16) according to any one of the preceding claims, **characterized in that** the nacelle (32) comprises at least two bearing members (64) spaced apart angularly around the insertion axis (A-A'), the adapter (39) comprising a retaining surface (49) for each bearing surface (64).

11. The device (16) according to any one of the preceding claims, **characterized in that** the or each bearing member (64) is formed by a rim perpendicular to the insertion axis (A-A') protruding radially away from the container (60).

12. The device (16) according to any one of the preceding claims, **characterized in that** the container (60) comprises a bottom wall (68) bearing a culture surface and a side wall (70) delimiting an upper opening (73), the nacelle (32) delimiting at least one passage (74) for supplying the culture surface with culture medium, separate from the upper opening (73).

13. A cell or tissue culture assembly (10), **characterized in that** it comprises:
- a tray (12) having at least one cavity (20) designed to receive a culture medium (14);
- a device (16) according to any one of the preceding claims, the support (30) being mounted on the tray (12) to place the reception opening (38) across from the cavity (20).

14. The assembly (10) according to claim 13, **characterized in that** the cavity (20) delimits a culture medium receiving well, the well emerging by an upper opening (21) with a contour similar to the contour of the reception opening (38).
